(19) Europäisches Patentamt
European Patent Office
Office européen des brevets

(11) Publication number: **0 070 657**
**B1**

(12) **EUROPEAN PATENT SPECIFICATION**

(45) Date of publication of patent specification: **04.12.85**

(51) Int. Cl.$^4$: **C 01 B 33/28,** B 01 J 29/28

(21) Application number: **82303607.4**

(22) Date of filing: **09.07.82**

(54) **Transition element-containing crystalline aluminosilicate composition.**

(30) Priority: **14.07.81 JP 109869/81**

(43) Date of publication of application:
**26.01.83 Bulletin 83/04**

(45) Publication of the grant of the patent:
**04.12.85 Bulletin 85/49**

(84) Designated Contracting States:
**DE FR GB IT NL**

(56) References cited:
**EP-A-0 031 255**
**GB-A-2 033 358**
**US-A-3 575 887**

**The file contains technical information submitted after the application was filed and not included in this specification**

(73) Proprietor: **MITSUBISHI GAS CHEMICAL COMPANY, INC.**
**5-2, Marunouchi 2-chome Chiyoda-Ku**
**Tokyo (JP)**

(72) Inventor: **Suzuki, Takashi**
**1-4248, Tsukefune-cho**
**Niigata-shi Niigata-ken (JP)**
Inventor: **Hashimoto, Shoichiro**
**1741-4, Tayuhama**
**Niigata-shi Niigata-ken (JP)**
Inventor: **Nakano, Rieko**
**4-13-9, Higashinakajima**
**Niigata-shi Niigata-ken (JP)**
Inventor: **Orisaka, Masami**
**4-19, Takara-machi**
**Niigata-shi Niigata-ken (JP)**

(74) Representative: **Weisert, Annekäte, Dipl.-Ing. Dr.-Ing. et al**
**Patentanwälte Kraus Weisert & Partner Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 070 657 B1

**Description**

This invention relates to a novel crystalline aluminosilicate composition. More specifically, this invention relates to a novel crystalline aluminosilicate containing a transition element and having very good properties as an adsorbent or catalyst.

Crystalline aluminosilicates are porous substances generally known as zeolites. They include, for example, naturally occurring zeolites such as mordenite, montmorillonite and clinoptilonite and synthetic zeolites A, X, Y, etc. produced by hydro-thermally reacting an aqueous solution containing a silicon compound and an aluminum compound.

Recently, synthetic zeolites having different skeletal structures from natural zeolites, such as ZSM-5 (Japanese Patent Publication No. 10,064/1971), ZSM-38 (Japanese Laid-Open Patent Publication No. 120,292/1977) and ZSM-34 (Japanese Laid-Open Patent Publication No. 58,499/1978), were suggested. These synthetic zeolites are produced by hydrothermal reaction in the presence of organic cations. For example, ZSM-5 is produced by hydrothermal reaction in the presence of a tetrapropylammonium cation. This method makes it possible to form a skeletal structure containing $SiO_2$ and $Al_2O_3$ in a wide range of mole ratios.

Since these porous crystalline aluminosilicates have the property of selecting the shape of a molecule, they find a wide range of applications as adsorbents or catalysts. For example, they are actually used as dehydrating agents for organic materials or as isomerization catalysts for hydrocarbons.

Furthermore fluoride containing crystalline aluminosilicates are known which are composited with a hydrogenative metal component such as metals of group VI B (Cr, Mo or W) and group VIII of the Periodic Table and mixtures thereof (US—A—3575887).

Some of the present inventors previously disclosed that a novel crystalline aluminosilicate zeolite having a unique crystal form and optionally containing fluorine can be provided by hydrothermally reacting zeolite in the presence of a fluorine ion, and that by exposing the acidic site through treatment with hydrochloric acid or otherwise, the resulting crystalline aluminosilicate can be used as a solid acid catalyst for various reactions such as the isomerization, disproportionation or alkylation of hydrocarbons, and the production of olefins, paraffins and aromatic compounds from methanol European Laid-Open Patent Publication No. 0031255).

It is an object of this invention to provide a novel crystalline aluminosilicate composition.

Another object of this invention is to provide a novel crystalline aluminosilicate composition containing a transition element.

Still another object of this invention is to provide a novel, transition element-containing, crystalline aluminosilicate composition having better properties than the transition metal-free crystalline aluminosilicate previously proposed by some of the present inventors.

Yet another object of this invention is to provide a novel crystalline aluminosilicate composition whose regeneration or activation after use as a catalyst can be easily effected even at low temperature of, for example, about 380°C which is much lower than the spontaneous ignition point (about 530°C) of tar deposited on the catalyst.

Further objects and advantages of this invention will become apparent from the following description.

These objects and advantages are achieved in accordance with this invention by a transition element-containing crystalline aluminosilicate composition having seven diffraction lines showing the following interplanar spacings and relative intensities in its X-ray diffraction pattern:

| Interplanar spacing (Å) | Relative intensity |
|---|---|
| 11.2 ± 0.4 | not more than 30 |
| 10.0 ± 0.4 | 30—100 |
| 5.01 ± 0.1 | 5—40 |
| 3.83 ± 0.05 | 30—100 |
| 3.75 ± 0.05 | 5—40 |
| 3.72 ± 0.05 | not more than 40 |
| 3.65 ± 0.05 | not more than 30 |

taking the intensity of the diffraction line at an interplanar spacing of 10.0 ± 0.4 or 3.83 ± 0.05 Å as 100.

A crystalline aluminosilicate composition in accordance with this invention contains a transition element and occasionally a fluorine atom.

The X-ray diffraction pattern is obtained by a standard powder diffraction method using $CuK_\alpha$ radiation of an X-ray diffractometer such as a self-recording X-ray diffractometer (GEIGERFLEX Model DS manufactured by Rigaku Denki Co., Japan).

The absolute value of the diffraction angle of each X-ray diffraction line obtained by this method may vary within a small tolerable range depending upon the method and conditions for sample preparation, measurement errors, etc. The values ± 0.4 — ± 0.05 in the interplanar spacings correspond to the values ± 0.3° in the diffraction angles (2θ), and show such a range of variations.

The transition element-containing crystalline aluminosilicate composition of this invention has within the diffraction angle range of 7 to 25° at least seven diffraction lines which can be clearly distinguished from each other and are characterized by having the relative intensities indicated hereinabove. In no case, the number of diffraction lines becomes less than seven by the overlapping of these diffraction lines, for example by the overlapping of two diffraction lines to appear as a single diffraction line.

Typically, the aforesaid seven diffraction lines of the transition element-containing crystalline aluminosilicate composition of this invention are expressed by the following interplanar spacings (Å) and relative intensities.

| Interplanar spacing (Å) | Relative intensity |
|---|---|
| 11.2 | not more than 30 |
| 10.0 | 30—100 |
| 5.01 | 5—40 |
| 3.83 | 30—100 |
| 3.75 | 5—40 |
| 3.72 | not more than 40 |
| 3.65 | not more than 30 |

The Bragg's equation may be used to obtain the interplanar spacings (Å) of a crystal from the diffraction angles (2θ) of X-ray diffraction lines.

The relationships between the diffraction angles (2θ) and the interplanar spacings (Å) of the seven diffraction lines determined by the Bragg's equation are as follows:

| Diffraction angle (2θ, degrees) | Interplanar spacing (Å) |
|---|---|
| 7.9 ± 0.3 | 11.2 ± 0.4 |
| 8.8 ± 0.3 | 10.0 ± 0.4 |
| 17.7 ± 0.3 | 5.01 ± 0.1 |
| 23.2 ± 0.3 | 3.83 ± 0.05 |
| 23.7 ± 0.3 | 3.75 ± 0.05 |
| 23.9 ± 0.3 | 3.72 ± 0.05 |
| 24.4 ± 0.3 | 3.65 ± 0.05 |

The crystalline aluminosilicate composition of this invention has crystallographic properties characterized by having the above-mentioned X-ray diffraction pattern. In terms of chemical composition, it is characterized by containing a transition element and occasionally fluorine.

The transition element, as referred to in the present application, denotes elements having atomic numbers 21—29, 39—47, 57—79 and 89 or more in the periodic table.

For example, the composition of this invention used as a catalyst preferably contains such a transition element as vanadium, chromium, manganese, iron, cobalt, nickel, copper, molybdenum, ruthenium, rhodium, palladium, silver, tungsten, platinum, or gold.

The composition of this invention may contain a transition element in the form of a metal, a compound, or an ion.

The composition of this invention contains such a transition element in an amount of preferably about 0.07 to about 80% by weight, especially about 0.2 to about 60% by weight, above all about 0.4 to about 40% by weight.

The composition of this invention can be produced by including a transition element compound or a transition metal in a starting aqueous mixture for the production of a crystalline aluminosilicate. Specifically, it can be produced by hydrothermally reacting a starting aqueous mixture containing a silicon compound, an aluminum compound, a fluorine compound, a compound yielding an organic cation in water and an alkali in the presence of a transition element compound or a transition metal.

The transition element compound may be water-soluble or water-insoluble. Water-soluble transition element compounds include compounds yielding an anion containing a transition element in water, for example such acids as vanadic acid, molybdic acid or tungstic acid and salts of these; and compounds yielding a transition element cation in water, such as copper sulfate, cobalt acetate or nickel nitrate. Water-insoluble transition element compounds include, for example, oxides of transition elements such as vanadium pentoxide, chromium oxide, copper oxide and nickel oxide. Transition element hydroxides in gel form such as iron hydroxide or cobalt hydroxide gel can also be used as the transition element compound.

Colloidal transition metals such as colloidal palladium, colloidal silver and colloidal platinum are preferred as the transition metal.

Sodium silicate, solid silica, silica gel, silica sol, etc. generally employed as silica sources in hydrothermal synthesis reaction may be used as the silicon compound. Silica sol is preferred, and for example, LUDOX (a tradename for a product of E. I. du Pont de Nemours & Co.), SNOWTEX (a tradename for a product of Nissan Chemical Co., Ltd.), and CATALOID (a tradename for a product of Catalytic Chemical Co., Ltd.) can be suitably used.

Sodium aluminate, freshly prepared aluminium hydroxide, etc. may be used as the aluminum compound.

The fluorine compound may be any compound which yields a fluorine ion in the reaction system. For example, there can be used not only those compounds which yield a fluorine ion when dissolved in water, such as sodium fluoride and ammonium fluoride, but also those which yield a fluorine ion when hydrolyzed, such as silicon fluoride, boron fluoride and sulfur fluoride. The fluorine compounds which yield a fluorine ion when dissolved in water, especially sodium fluoride and ammonium fluoride, are preferred.

Sodium compounds, especially sodium hydroxide, are suitably used as the alkali.

When sodium aluminate is used as the aluminum compound, it also acts as an alkali metal compound.

For practical purposes, quaternary ammonium cations are advantageously used as the organic cation. Such quaternary ammonium ions are represented, for example, by the following formula (1)

$$R_4N^+ \quad (1)$$

wherein R represents a lower alkyl group. Preferably, R is an alkyl group having 1 to 4 carbon atoms, such as methyl, ethyl, propyl and butyl. Examples of such quaternary ammonium ions are tetramethylammonium, tetraethylammonium, tetrapropylammonium and tetrabutylammonium ions.

The quaternary ammonium ions of the above formula (1) are derived from quaternary ammonium compounds of the following formula (2)

$$R_4NX \quad (2)$$

wherein R is as defined for formula (1), and X represents a halogen atom such as chlorine, bromine or iodine, a residue of an acid such as a sulfuric acid radical, or an anion such as a hydroxyl group. Or they can be obtained by forming the quaternary ammonium compounds of formula (2) in the reaction system by using both tertiary amines of the following formula (3)

$$R_3N \quad (3)$$

wherein R is as defined for formula (1) and compounds of the following formula (4)

$$RX \quad (4)$$

wherein R and X are as defined for formula (2).

Specific examples of the quaternary ammonium compounds of formula (2) include tetrapropylammonium bromide, tetramethylammonium bromide, tetraethylammonium bromide, tetrapropylammonium chloride, tetrabutylammonium bromide and tetrabutylammonium chloride; and tetramethylammonium, tetraethylammonium, tetrapropylammonium and tetrabutylammonium hydroxides and sulfates.

Examples of the tertiary amines of formula (3) are trimethylamine, triethylamine, tripropylamine and tributylamine.

Specific examples of the compounds of formula (4) are methyl bromide, ethyl bromide, propyl bromide, propyl chloride, butyl bromide, butyl chloride, methyl alcohol, ethyl alcohol, dimethyl sulfate and diethyl sulfate.

The aforesaid starting compounds are used in mole ratios of the following ranges.

| | Usual range (mole ratios) | Preferred range (mole ratios) | Most Preferred range (mole ratios) |
|---|---|---|---|
| $SiO_2/Al_2O_3$ | 50 or more | 100—3000 | 200—2000 |
| $R_4N^+/SiO_2$ | 0.1 or more | 0.1—1.0 | 0.2—0.8 |
| $Na^+/SiO_2$ | 0.1—0.8 | 0.15—0.7 | 0.2—0.6 |
| $F^-/SiO_2$ | 0.1 or more | 0.1—1.0 | 0.2—0.8 |
| $H_2O/SiO_2$ | 10 or more | 10—1000 | 30—70 |
| $M/SiO_2$ | 0.001 or more | 0.003—0.3 | 0.005—0.2 |

In the above table, the silicon compound is expressed as $SiO_2$, and the aluminum compound, as $Al_2O_3$. $R_4N^+$, $Na^+$, and $F^-$ respectively represent the organic cation, alkali and fluorine compound. M represents a transition element, and $M/SiO_2$represents M(atom) $SiO_2$ (mole).

Commercially available silica gel or silica sol usually contains about 1000 ppm of $Al_2O_3$, and may give a crystalline aluminosilicate without particularly adding another aluminum compound.

The $SiO_2/Al_2O_3$ mole ratio in the starting mixture will approximately be the $SiO_2/Al_2O_3$ mole ratio in the resulting crystalline aluminosilicate.

Preferably, the aqueous starting mixture is subjected to shear mixing prior to submitting it to hydrothermal reaction because this results in thorough mixing of the starting mixture. The shear mixing denotes such an extent of mixing as to generate a shear stress in the aqueous starting mixture. This can be achieved, for example, by using a high-speed rotary mixer, a homogenizer, a colloid mill, etc. When such mixing machines are used, it is preferred that the linear velocity of the edge tip of a rotating portion of such a machine be generally adjusted to at least 10 m/sec.

The hydrothermal reaction can be carried out by heating the aqueous starting mixture to a temperature of 100 to 200°C, preferably 130 to 170°C. Preferably, the reaction is carried out under autogenous pressures, and it is desirable to adjust the rate of temperature elevation to at least 1°C/min., especially to at least 2°C/min. The reaction time is generally 4 hours to 150 hours.

The transition element-containing crystalline aluminosilicate produced by the above method can be obtained by cooling the reaction mixture to room temperature, filtering it, washing the filtrate with water, and then separating the washed product.

Alternatively, the transition metal-containing crystalline aluminosilicate composition of this invention may be produced by (i) depositing a transition element cation on a crystalline aluminosilicate not containing a transition element by ion exchange, (ii) impregnating the transition element-free crystalline aluminosilicate with an aqueous solution of a transition element compound and then drying and calcining the impregnated aluminosilicate, or (iii) mechanically mixing the transition element-free crystalline aluminosilicate with a powder of a transition metal or transition element compound.

The transition metal-free crystalline aluminosilicate (to be referred to as FZ-1 zeolite) used in these alternative procedures can be produced in a similar way to the process of producing the composition of this invention described in detail hereinabove except that a starting aqueous mixture not containing a transition metal nor a transition element compound is used instead of the aforesaid starting aqueous mixture used in hydrothermal reaction.

The resulting FZ-1 zeolite contains fluorine. It is usually dried at a temperature of, for example, 100 to 120°C, and then calcined at a temperature of, for example, 350 to 800°C, preferably 400 to 600°C, for 2 to 24 hours, preferably 4 to 10 hours. The FZ-1 zeolite dried and calcined under these conditions can be suitably used as the starting crystalline aluminosilicate in the above-mentioned alternative procedures. As necessary, the dried and calcined FZ-1 zeolite may be treated with an acidic solution of at least one inorganic acid such as hydrochloric acid, sulfuric acid or nitric acid or at least one organic acid such as formic acid, oxalic acid, acetic acid or citric acid, and as necessary, further calcined. Preferably, the acid treatment is carried out by using a 1—22% by weight aqueous solution of hydrochloric acid at a temperature of 10 to 120°C, especially 40 to 100°C, for a period of 0.1 to 30 hours, especially 1 to 10 hours.

The FZ-1 zeolite ($H^+$ form) which has undergone such additional acid treatment and optional calcination can also be used as the starting crystalline aluminosilicate in the alternative procedures.

The fluorine of the FZ-1 zeolite may decrease in amount or disappear during the calcination or acid treatment under the aforesaid conditions.

The alternative procedure (i) can be practiced by coating a powder of the starting crystalline aluminosilicate (having a particle diameter of, for example, 1 to 100 microns) with an aqueous solution of a transition element compound which is water-soluble and yields a transition element cation in water, and

washing the resulting product with water. Since according to this procedure, the amount of the transition element which can be deposited by ion exchange depends on the $Al_2O_3$ content of the FZ-1 zeolite used, it is impossible to include so large an amount of the transition element, but in the resulting composition of this invention, the dispersibility of the transition element is good.

The alternative procedure (ii) can be practiced by impregnating a powder of the crystalline aluminosilicate, similar to the above, with an aqueous solution of such a water-soluble transition element compound as mentioned above, and drying and calcining the impregnated aluminosilicate. The drying and calcination can be performed under the same conditions as described hereinabove with regard to the FZ-1 zeolite. According to this procedure, the maximum amount of the transition element which can be included into the crystalline aluminosilicate composition of this invention is restricted by the solubility of the transition element compound used. Nevertheless, it is possible to produce a crystalline aluminosilicate composition containing a fairly large amount of the transition element.

According to the alternative procedures (i) and (ii), the composition of this invention can be advantageously produced by using the transition element compound such that the proportion of the transition element is at least 0.001 atom, preferably 0.003 to 0.3 atom, per mole of $SiO_2$ in the FZ-1 zeolite.

The alternative procedure (iii) can be practiced by simply mechanically mixing a powder of a transition metal or a transition element compound (having a particle diameter of, for example, 0.1 to 100 microns) with a powder of the FZ-1 zeolite. The greatest characteristic of this procedure is that both a transition metal and a transition element compound can be used. It also has the advantage that the content of the transition element can be varied over a wide range, and substantially all of the transition element used can be included into the resultant composition.

A preferred composition of this invention can be provided by using the transition metal or the transition element compound such that the proportion of the transition element is at least 0.001 atom, especially 0.003 to 1 atom, per mole of $SiO_2$ in the FZ-1 zeolite.

The composition of this invention can be produced by the various manufacturing methods described above, and all of the products are those in which the transition element is taken up in the crystal structure of FZ-1 zeolite without substantially disturbing the crystal structure, and/or mixtures of FZ-1 zeolite and a transition element (metal or compound), which are characterized by the aforesaid X-ray diffraction pattern.

The transition element-containing crystalline aluminosilicate composition of this invention can be suitably used as a catalyst for various reactions, an adsorbent, etc.

The catalyst or adsorbent may be in various forms such as a finely divided powder, pellets, tablets and an extrusion-molded article. Or the composition of this invention may be molded in the wet or dry state after mixing it with a refractory inorganic oxide, such as clay, alumina, silica, silica-alumina or titania, which is usually employed as a binder for zeolite catalysts. In this case, the content of the binder is desirably 1 to 99% by weight, preferably 10 to 80% by weight. The molding may also be performed after further adding ligninsulfonic acid, stearic acid, starch, polyvinyl alcohol, or crystalline cellulose in addition to the binder. The resulting zeolite molded articles may be calcined as required, and can be used as a catalyst, an adsorbent, etc.

The transition element-containing crystalline aluminosilicate composition of this invention has the advantage that when it is used as a catalyst, the temperature required to regenerate the used catalyst can be decreased to about 380°C. A conventional crystalline aluminosilicate not containing a transition element, such as a catalyst containing FZ-1 zeolite, after it has been used for a long period of time as a catalyst, needs to be regenerated or activated by burning off the tarry matter deposited on its surface under heat in an atmosphere of air. The temperature required for this regeneration procedure is higher than about 530°C which is the spontaneous ignition point of the tarry material.

Since the composition of this invention used as a catalyst can be regenerated at a lower regeneration temperature than those temperatures used for regenerating conventional aluminosilicate catalysts, the time required for regeneration can be shortened, and a metallic and a non-metallic material used for reactors can be easily selected. Hence, such a catalyst has very advantageous utility in various practical applications.

The transition element-containing composition of this invention exhibits catalytic activity in many reactions, such as the conversion, decomposition, oxidation and reduction of hydrocarbons and carbon monoxide, and can be suitably utilized therefor.

The following examples illustrate the present invention more specifically.

## Example 1

3.3 g of sodium aluminate (34.9% by weight as $Al_2O_3$ and 33.3% by weight as $Na_2O$), 1.27 g of sodium hydroxide and 1.19 g of ammonium fluoride were dissolved in 146 g of water, and 10.7 g of tetrapropylammonium bromide was further added to form a uniform aqueous solution. Furthermore, 31.5 g of 30% by weight silica sol and 1.0 g of nickel acetate tetrahydrate were added to the aqueous solution. The mixture was put in a mixer and worked up for 3 minutes at room temperature at a rotating speed of 10,000 rpm (the linear speed of the edge tip being 45 m/sec.). The starting mixture was then put in a corrosion-resistant pressure vessel, and heated to 150°C over 1 hour and maintained at this temperature for 62 hours under autogenous pressure. The resulting reaction product was then cooled, and the resulting

crystals were collected by filtration. The crystals were washed with water until the washing became neutral. The washed crystals were dried at 110°C for 10 hours.

The mole ratios of the components of the starting mixture, the hydrothermal reaction conditions, and the size, X-ray diffraction line data, fluorine content and transition element content of the resulting crystals are summarized in Table 1.

Examples 2 to 7

The same hydrothermal reaction as in Example 1 was carried out except that the mole ratios of the starting materials, the type of the transition element and the reaction time were varied as shown in Table 1. The results are shown in Table 1.

Comparative Example 1

The same hydrothermal reaction as in Example 1 was carried out except that no transition element was used. FZ-1 zeolite (containing fluorine) was obtained. The reaction conditions and the results are shown in Table 1.

| | | Example 1 | Example 2 | Example 3 | Example 4 |
|---|---|---|---|---|---|
| Mole ratios of the starting materials charged | $SiO_2/Al_2O_3$ | 411 | 445 | 407 | 445 |
| | $Na^+/SiO_2$ | 0.23 | 0.22 | 0.23 | 0.23 |
| | $R^+/SiO_2$ | 0.25 | 0.25 | 0.25 | 0.25 |
| | $F^-/SiO_2$ | 0.20 | 0.20 | 0.20 | 0.20 |
| | $H_2O/SiO_2$ | 59.7 | 59.8 | 58.9 | 59.6 |
| | $M/SiO_2$ | 0.025 | 0.007 | 0.025 | 0.06 |
| Transition metal compound charged | | $Ni(CH_3COO)_2 \cdot 4H_2O$ | $(NH_4)_{10}W_{12}O_{41} \cdot 5H_2O$ | $Cr_2O_3$ | $Cr_2O_3$ |
| Reaction temperature | (°C) | 150 | 150 | 150 | 150 |
| Reaction time | (hr.) | 62 | 61 | 62 | 62 |
| Size of the crystals (*1) | (μm) | 30 × 10 | 30 × 13 | 30 × 12 | 30 × 12 |
| X-ray diffraction line data of the crystals (*2) | 11.3 | 13 | 9 | 10 | 12 |
| | 10.0 | 70 | 44 | 47 | 29 |
| | 5.01 | 18 | 10 | 11 | 9 |
| | 3.84 | 100 | 100 | 100 | 100 |
| | 3.75 | 26 | 25 | 25 | 29 |
| | 3.72 | 21 | 19 | 20 | 19 |
| | 3.65 | 16 | 13 | 14 | 13 |
| Fluorine content of the crystals (wt. %) | | 0.6 | 0.6 | 0.6 | 0.6 |
| Transition element content of the crystals (wt. %) | | 2.0 | 1.0 | 0.6 | 1.4 |

TABLE 1 (Continued)

| | | Example 5 | Example 6 | Example 7 | Comp. Ex. 1 |
|---|---|---|---|---|---|
| Mole ratios of the starting materials charged | $SiO_2/Al_2O_3$ | 476 | 445 | 401 | 454 |
| | $Na^+/SiO_2$ | 0.22 | 0.21 | 0.22 | 0.22 |
| | $R^+/SiO_2$ | 0.28 | 0.28 | 0.25 | 0.28 |
| | $F^-/SiO_2$ | 0.33 | 0.32 | 0.20 | 0.21 |
| | $H_2O/SiO_2$ | 59.1 | 59.2 | 59.6 | 58.9 |
| | $M/SiO_2$ | 0.001 | 0.034 | 0.032 | 0 |
| Transition metal compound charged | | $Fe(NO_3)_3.9H_2O$ | $6TiO_2.3/2H_2O$ | $(NH_4)_6Mo_7O_{24}.4H_2O$ | – |
| Reaction temperature | (°C) | 150 | 150 | 150 | 150 |
| Reaction time | (hr.) | 65 | 65 | 63 | 63 |
| Size of the crystals (*1) | (μm) | 68 × 16 | 50 × 10 | 33 × 12 | 45 × 20 |
| X-ray diffraction line data of the crystals (*2) | 11.3 | 14 | 4 | 12 | 7 |
| | 10.0 | 91 | 100 | 78 | 100 |
| | 5.01 | 23 | 26 | 19 | 24 |
| | 3.84 | 100 | 62 | 100 | 54 |
| | 3.75 | 23 | 18 | 27 | 15 |
| | 3.72 | 25 | 10 | 20 | 13 |
| | 3.65 | 15 | 6 | 15 | 9 |
| Fluorine content of the crystals (wt. %) | | 0.9 | 0.9 | 0.6 | 0.6 |
| Transition element content of the crystals (wt. %) | | 0.1 | 3.0 | 3.4 | 0 |

Note to Table 1

(*1): Shown by the length of the longest edge x the length of the shortest edge.

(*2): Relative intensities at interplanar spacings (Å) are shown. For example, in the crystals of Examples 1 to 5 and 7, an X-ray diffraction line corresponding to an interplanar spacing of 3.84 Å shows the highest intensity (100). Likewise, in the crystals of Example 6 and Comparative Example 1, an X-ray diffraction line corresponding to an interplanar spacing of 10.0 Å shows the highest intensity (100).

Referential Example 1

The transition element-containing crystalline aluminosilicate composition obtained in Example 1 in powder form was calcined in the air at 550°C for 5 hours. Then, the calcined product was treated with an 18% by weight aqueous solution of hydrochloric acid at 90°C for 5 hours, cooled, filtered, and washed with water until no chlorine ion was detected in the washing. The washed product was dried at 110°C for 10 hours. The resulting powder was well kneaded with 20% by weight silica sol, and the mixture was moled by using a porous plate (3 mm in diameter × 3 mm in height). After drying, the molded article was taken out from the porous plate, and calcined in the air at 550°C for 5 hours. The calcined product was subjected to ion exchange treatment at 90°C for 5 hours using a 10% by weight aqueous solution of ammonium chloride. The product was cooled, filtered, washed with water, and dried at 110°C for 10 hours. The dried product was further calcined at 550°C for 4 hours in the air for use as a catalyst. This catalyst contained 80% by weight of an aluminosilicate composition and 20% by weight of silica as a carrier. The resulting catalyst (4.4 ml) was filled in a reaction tube having a sectional area of 7.3 $cm^2$ to a height of 0.6 cm. Methanol was passed through the catalyst bed kept at 335°C at a velocity (LSHV) of 1.2/hr without using a diluent to produce hydrocarbons. The results of the reaction at 5 hours and 70 hours from the start of the reaction are shown in Table 2.

After performing the reaction for 80 hours, the catalyst was regenerated in the air at 450°C for 5 hours, or at 380°C for 5 hours. Then, methanol was again fed and reacted. The results obtained are also shown in Table 2.

Referential Examples 2 to 4

Methanol was reacted to produce hydrocarbons in the same way as in Referential Example 1 by using the transition element-containing crystalline aluminosilicate compositions in powder form obtained in Examples 2 to 4, respectively. The results of the reactions are shown in Table 2.

The results of Referential Examples 1 to 4 show that the catalyst was well regenerated or activated even under relatively mild regeneration conditions involving a temperature of 380°C and a period of 5 hours.

Example 8

(1) The FZ-1 zeolite powder not containing a transition element, obtained in Comparative Example 1, was calcined in the air at 550°C for 5 hours. The calcined product was treated with an 18% by weight aqueous solution of hydrochloric acid at 90°C for 5 hours, cooled, filtered, and washed with water until no chlorine ion was detected in the washing. The washed product was dried at 110°C for 10 hours. Chromium oxide ($Cr_2O_3$) powder was added to the resulting $H^+$-form FZ-1 zeolite, and they were well mixed mechanically to give a crystalline aluminosilicate composition containing 2.0% by weight of chromium.

(2) 20% by weight silica sol was added to the resulting crystalline aluminosilicate composition, and they were well mixed. The mixture was molded by using a porous plate (3 mm Ø × 3 mmH). The molded article was worked up in the same way as in Referential Example 1 to prepare a catalyst. Methanol was passed through the catalyst to produce hydrocarbons. The results of the reaction are shown in Table 2. The used catalyst was well regenerated or activated when heated in an air atmosphere at 450°C for 5 hours. It could be regenerated even by heating at 380°C for 5 hours.

Comparative Referential Example

A catalyst was prepared in the same way as in Example 8, (2) using the transition element-free FZ-1 zeolite powder obtained in Comparative Example 1. Similarly, methanol was passed through the resulting catalyst to produce hydrocarbons. The results are shown in Table 2.

The results show that when the spent catalyst was heated at 450°C and 380°C, its regeneration was insufficient as compared with any of the catalysts shown in Referential Examples 1 to 4 and Example 8, (2).

TABLE 2

| | | Catalyst (the parenthesized matter shows the catalyst regenerating conditions in air) | 5 hours from the start | | |
|---|---|---|---|---|---|
| | | | Methanol conversion (%) | Selectivity for hydro-carbon (wt. %) | Selectivity for dimethyl ether (wt. %) |
| Referential Example | 1 | Fresh | 95 | 43 | 2 |
| | | Regenerated (450°C × 5hr) | 95 | 43 | 2 |
| | | Regenerated (380°C × 5hr) | 94 | 42 | 3 |
| | 2 | Fresh | 98 | 43 | 1 |
| | | Regenerated (450°C × 5hr) | 98 | 43 | 1 |
| | | Regenerated (380°C × 5hr) | 96 | 42 | 2 |
| | 3 | Fresh | 98 | 43 | 1 |
| | | Regenerated (450°C × 5 hr) | 98 | 43 | 1 |
| | | Regenerated (380°C × 5hr) | 96 | 43 | 1 |
| | 4 | Fresh | 99 | 43 | 1 |
| | | Regenerated (450°C × 5hr) | 99 | 43 | 1 |
| | | Regenerated (380°C × 5hr) | 95 | 41 | 4 |
| Example 8, (2) | | Fresh | 99 | 43 | 1 |
| | | Regenerated (450°C × 5hr) | 99 | 43 | 1 |
| | | Regenerated (380°C × 5hr) | 94 | 41 | 5 |
| Comparative Referential Example | | Fresh | 99 | 43 | 1 |
| | | Regenerated (450°C × 5hr) | 99 | 43 | 1 |
| | | Regenerated (380°C × 5hr) | 80 | 21 | 36 |

TABLE 2 (Continued)

| | | Catalyst (the parenthesized matter shows the catalyst regenerating conditions in air) | 70 hours from the start | | |
|---|---|---|---|---|---|
| | | | Methanol conversion (%) | Selectivity for hydro-carbon (wt. %) | Selectivity for dimethyl ether (wt. %) |
| Referential Example | 1 | Fresh | 90 | 38 | 9 |
| | | Regenerated (450°C × 5hr) | 90 | 38 | 9 |
| | | Regenerated (380°C × 5hr) | 89 | 37 | 11 |
| | 2 | Fresh | 92 | 39 | 8 |
| | | Regenerated (450°C × 5hr) | 92 | 39 | 8 |
| | | Regenerated (380°C × 5hr) | 90 | 38 | 9 |
| | 3 | Fresh | 91 | 40 | 6 |
| | | Regenerated (450°C × 5hr) | 91 | 40 | 6 |
| | | Regenerated (380°C × 5hr) | 90 | 38 | 9 |
| | 4 | Fresh | 91 | 39 | 8 |
| | | Regenerated (450°C × 5hr) | 90 | 38 | 9 |
| | | Regenerated (380°C × 5hr) | 90 | 38 | 9 |
| Example 8, (2) | | Fresh | 92 | 39 | 8 |
| | | Regenerated (450°C × 5hr) | 90 | 38 | 9 |
| | | Regenerated (380°C × 5hr) | 87 | 35 | 14 |
| Comparative Referential Example | | Fresh | 93 | 39 | 8 |
| | | Regenerated (450°C × 5hr) | 83 | 29 | 26 |
| | | Regenerated (380°C × 5hr) | 62 | 0 | 72 |

**Claims**

1. A transition element-containing crystalline aluminosilicate having seven diffraction lines showing the following interplanar spacings and relative intensities in its X-ray diffraction pattern:

| Interplanar spacing (Å) | Relative intensity |
|---|---|
| 11.2 ± 0.4 | not more than 30 |
| 10.0 ± 0.4 | 30—100 |
| 5.01 ± 0.1 | 5—40 |
| 3.83 ± 0.05 | 30—100 |
| 3.75 ± 0.05 | 5—40 |
| 3.72 ± 0.05 | not more than 40 |
| 3.65 ± 0.05 | not more than 30 |

taking the relative intensity of the diffraction line at an interplanar spacing of 10.0 ± 0.4 or 3.83 ± 0.05 Å as 100.

2. The composition of claim 1 which further contains a fluorine element.

3. The composition of claim 1 or 2 wherein the transition element is selected from elements having an atomic number of 21 to 29, 39 to 47, 57 to 79 and 89 or more in the periodic table.

4. The composition of claim 3 wherein the transition element is at least one element selected from group consisting of vanadium, chromium, manganese, iron, cobalt, nickel, copper, molybdenum, ruthenium, rhodium, palladium, silver, tungsten, platinum and gold.

5. The composition of claim 1 or 2 wherein the amount of the transition element is at least 0.001 atom per mole of $SiO_2$.

6. The composition of claim 5 wherein the amount of the transition element is 0.003 to 1 atom per mole of $SiO_2$.

7. The composition of claim 1 or 2 wherein the $SiO_2$:$Al_2O_3$ mole ratio is 50 or more:1.

8. The composition of claim 7 wherein the $SiO_2$:$Al_2O_3$ mole ratio is 100—3000:1.

**Patentansprüche**

1. Ein ein Übergangselement enthaltendes kristallines Aluminiumsilicat, welches sieben Diffraktionslinien aufweist, die die folgenden interplanaren Abstände und relativen Intensitäten im Röntgenbeugungsspektrum besitzen:

| Interplanar Abstand (Å) | Relative Intensität |
|---|---|
| 11,2 ± 0,4 | nicht mehr als 30 |
| 10,0 ± 0,4 | 30—100 |
| 5,01 ± 0,1 | 5—40 |
| 3,83 ± 0,05 | 30—100 |
| 3,75 ± 0,05 | 5—40 |
| 3,72 ± 0,05 | nicht mehr als 40 |
| 3,65 ± 0,05 | nicht mehr als 30 |

wobei die relative Intensität der Diffraktionslinie bei einem interplanaren Abstand von 10,0 ± 0,4 oder 3,83 ± 0,05 Å als 100 genommen wird.

2. Zusammensetzung nach Anspruch 1, dadurch gekennzeichnet, daß sie zusätzlich ein Fluorelement enthält.

3. Zusammensetzung nach Anspruch 1 oder 2, dadurch gekennzeichnet, daß das Übergangselement aus Elementen mit einer Atomzahl von 21 bis 29, 39 bis 47, 57 bis 79 und 89 oder mehr im Periodischen System ausgewählt wird.

4. Zusammensetzung nach Anspruch 3, dadurch gekennzeichnet, daß das Übergangselement mindestens ein Element aus der Gruppe Vanadium, Chrom, Mangan, Eisen, Kobalt, Nickel, Kupfer, Molybdän, Ruthenium, Rhodium, Palladium, Silber, Wolfram, Platin und Gold ist.

5. Zusammensetzung nach Anspruch 1 oder 2, dadurch gekennzeichnet, daß die Menge an Übergangselement mindestens 0,003 bis 1 Atom pro Mol $SiO_2$ beträgt.

6. Zusammensetzung nach Anspruch 5, dadurch gekennzeichnet, daß die Menge an Übergangselement 0,003 bis 1 Atom pro Mol $SiO_2$ beträgt.

7. Zusammensetzung nach Anspruch 1 oder 2, dadurch gekennzeichnet, daß das $SiO_2$—$Al_2O_3$—Molverhältnis 50 oder mehr zu 1 beträgt.

8. Zusammensetzung nach Anspruch 7, dadurch gekennzeichnet, daß das $SiO_2$-$Al_2O_3$-Molverhältnis 100 bis 3000 zu 1 beträgt.

**Revendications**

1. Un aluminosilicate cristallisé contenant un élément de transition, dont le spectre de diffraction de rayons X présente sept raies de diffraction avec les distances entre plans réticulaires et les intensités relatives suivantes:

| Distances entre plans réticulaires | Intensités relatives |
|---|---|
| 11,2 ± 0,4 | pas plus de 30 |
| 10,0 ± 0,4 | 30—100 |
| 5,01 ± 0,1 | 5—40 |
| 3,83 ± 0,05 | 30—100 |
| 3,75 ± 0,05 | 5—40 |
| 3,72 ± 0,05 | pas plus de 40 |
| 3,65 ± 0,05 | pas plus de 30 |

l'intensité relative de la raie de diffraction pour une distance entre plans réticulaires de 10,0 ± 0,4 ou 3,83 ± 0,05 Å étant prise égale à 100.

2. Composition selon la revendication 1 qui contient en outre du fluor.

3. Composition selon la revendication 1 ou 2 dans laquelle l'élément de transition est choisi parmi les élements de numéros atomiques 21 à 29, 39 à 47, 57 à 79 et 89 ou plus de la classification périodique.

4. Composition selon la revendication 3 dans laquelle l'élément de transition est constitué par un ou plusieurs éléments choisis parmi le vanadium, le chrome, le manganèse, le fer, le cobalt, le nickel, le cuivre, le molybdène, le ruthénium, le rhodium, le palladium, l'argent, le tungstène, le platine et l'or.

5. Composition selon la revendication 1 ou 2 dans laquelle la proportion de l'élément de transition est d'au moins 0,001 atome par mole de $SiO_2$.

6. Composition selon la revendication 5 dans laquelle la proportion de l'élément de transition est de 0,003 à 1 atome par mole de $SiO_2$.

7. Composition selon la revendication 1 ou 2 dans laquelle le rapport molaire $SiO_2:Al_2O_3$ est le rapport 50 ou un rapport plus élevé.

8. Composition selon la revendication 7 dans laquelle le rapport molaire $SiO_2:Al_2O_3$ est compris entre 100 et 3000.